(19) Europäisches Patentamt
European Patent Office
Office européen des brevets

(11) Publication number: **0 383 171 A2**

(12) **EUROPEAN PATENT APPLICATION**

(21) Application number: 90102366.3

(22) Date of filing: 07.02.90

(51) Int. Cl.5: C07H 15/256, A61K 31/705, C07C 62/30, C07C 35/44, C07C 43/02, C07C 69/757, C07C 235/40

Claims for the following Contracting States: ES + GR.

(30) Priority: 11.02.89 EP 89102392

(43) Date of publication of application: 22.08.90 Bulletin 90/34

(84) Designated Contracting States: AT BE CH DE DK ES FR GB GR IT LI LU NL SE

(71) Applicant: HOECHST AKTIENGESELLSCHAFT
Postfach 80 03 20
D-6230 Frankfurt am Main 80(DE)

(72) Inventor: De Souza, Noel, John Dr.
145 Niblana, Pali Hill
Bandra, Bombay 400 050(IN)
Inventor: Shah, Virbala
230B Panch Ratna, Opera House
Bombay 400 004(IN)
Inventor: Desai, Premanand Durgarao, Dr.
403 Sea Side Apartments, P. Balu Marg
Prabhadevi, Bombay 400025(IN)
Inventor: Inamdar, Prabhakar Krishnaji, Dr.
22 Kalpavriksha, Co.op.Hsg.Soc. Sant
Muktabai Marg
Vile Parle,(East), Bombay 400 057(IN)
Inventor: D'Sa, Adolf, Dr.
601 Miramar, Veer Savarkar Marg, Dadar
Bombay 400 028(IN)
Inventor: Ammanamanchi, Radhakrishna, Dr.
2-2-1167/11/1, Tilak Nagar
Hyderabad 500 044 Andhra Pradesh(IN)
Inventor: Dohadwalla, Alihussein Nomanbhai, Dr.
Noman Mansion 139 C., Cumballa Hill
August Kranti Marg, Bombay 400 036(IN)
Inventor: Lakdawala, Aftab Dawoodbhai, Dr.
11, Kapadia Mansion, Ground floor, Dr. A.Nair Road
Bombay 400 011(IN)
Inventor: Mandrekar, Sadashiv Shantaram
B, Darshan, Linking Road Extn., Santa Cruz (West)
Bombay 400 054(IN)
Inventor: Blumbach, Jürgen, Dr.
Nepean Sea Road, Nilandri
Bombay 400006(IN)

(54) **2,3,23-trihydroxy-urs-12-ene and its derivatives, processes for their preparation and their use.**

(57) 2,3,23-Trihydroxy-urs-12-ene and its derivatives of the formula

EP 0 383 171 A2

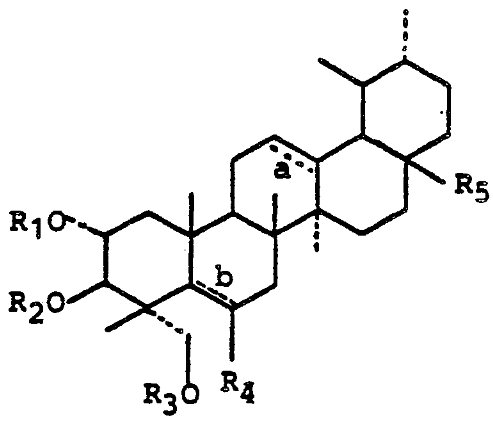

in which the substituents $R_1$ - $R_5$ have the given meaning can be used for the treatment and prophylaxis of cognitive disorders, cerebral vascular diseases and diseases of the central nervous system.

## 2,3,23-Trihydroxy-urs-12-ene end its derivatives, processes for their preparation and their use

The invention relates to 2,3,23-trihydroxy-urs-12-ene and its derivatives, processes for their preparation, and their use for the treatment of cognitive disorders, cerebral vascular diseases, and diseases of the central nervous system.

Among the most prominent human cognitive disorders are mental retardation, learning disabilities or the dementias (primary degenerative dementia PDD or Alzheimer's disease), mild or minimal memory improvement (benign senescent forgetfulness) or multi-infarct dementia (MID). No drugs have been clearly identified yet that are effective in the prevention or treatment of these disorders.

Different types of compounds are known to have cognition activating effects in animal models for cognitive disorders. Among the better known compounds are the nootropic agents which are mainly pyrrollidone derivatives such as piracetam, and the nitrogen-containing compounds that act on the cholinergic system to alleviate the cholinergic deficit in dementia, viz. muscarinic or nicotinic agents, cholinesterase inhibitors, cholinergic releasing agents. [Ref. F. M. Hersheusom, J. C. Mancott and W. H. Moss, Ann. Rep. Med. Chem. 21,31(1986)].

It has now been found that the administration of compounds belonging to the 2,3,23-trihydroxy-urs-12-ene series results in an improvement in impaired cognitive function in mammals and thus they are useful drugs for the prevention and treatment of cognitive disorders. The compounds of the invention also display inhibition of capillary permeability and antagonism of serotonin, properties which are useful for cerebral vascular diseases and in general for diseases of the central nervous system. While some of the compounds of the invention are known and are used as wound-healing agents, their usefulness in treating the disorders described above, viz. cognitive disorders, cerebral vascular diseases and diseases of the central nervous system, is not previously known.

The instant invention relates to 2,3,23-trihydroxy-urs-12-ene derivatives of the formula I

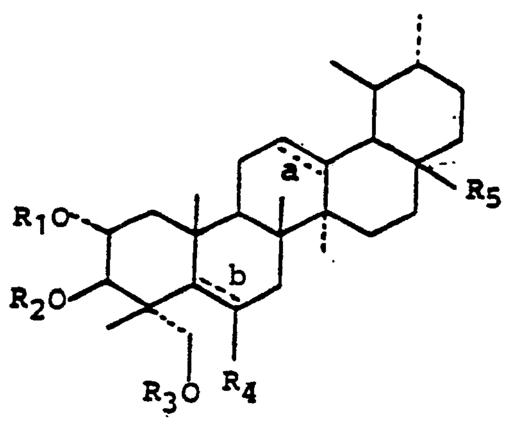

(I)

in which

$R_1$, $R_2$ and $R_3$ which may be the same or different stand for hydrogen, alkyl, aralkyl, acyl, substituted aminoacyl or mono- or polyglycosyl,

$R_4$ stands for hydrogen, hydroxy, alkoxy, aralkoxy, acyloxy or a mono- or polyglycosyloxyether group, 'b' symbolizes an optional additional bond between carbon atoms C5 and C6; $R_4$ also may stand for an oxo-group when 'b' does not symbolize an additional bond between C5 and C6,

$R_5$ stands for the group

$COOR_6$, wherein $R_6$ stands for hydrogen, alkyl, substituted alkyl or mono- or polyglycosyl,

or the group:

$CH_2OR_7$ wherein $R_7$ stands for hydrogen, alkyl, substituted alkyl, acyl or mono- or polyglycosyl,

or the group

$CONR_8R_9$ wherein $R_8$ and $R_9$ when they are the same, stand for hydrogen, alkyl or substituted alkyl; when $R_8$ stands for hydrogen, $R_9$ stands for alkyl, substituted alkyl or aryl; when $R_8$ and $R_9$ together with the nitrogen atom to which they are attached form a heterocycle, which may contain more than one heteroatom and is optionally substituted at one or more places by groups such as alkyl, hydroxy, alkoxy, aryl or another heterocyclic group and 'a' symbolizes an optional additional bond between carbon atoms C12 and C13,

with the exception of those compounds, in which at the same time $R_1$, $R_2$ and $R_3$ are H or $R_1$, $R_2$ and $R_3$ are acetyl, $R_4$ is H or OH and $R_5$ is COOH or $COOCH_3$ or in which at the same time $R_1$ and $R_3$ are acetyl, $R_2$ is H or $R_1$ is acetyl and $R_2$ and $R_3$ are H, $R_4$ is H and $R_5$ is COOH or $COOCH_3$ or in which at the same time $R_1$, $R_2$ and $R_3$ are H, $R_4$ is H or OH and $R_5$ is a O-6-deoxy-α-L-mannopyranosyl-(1→4)-O-β-D-glucopyranosyl-(1→6)-β-D-glucopyranosyl-oxycarbonyl-residue or in which at the same time $R_1$, $R_2$ and $R_4$ are H, $R_2$ is $CH_3$ and $R_5$ is $COOCH_3$.

Furthermore, the invention relates to 2,3,23-trihydroxy-urs-12-ene and its derivatives of the formula I′

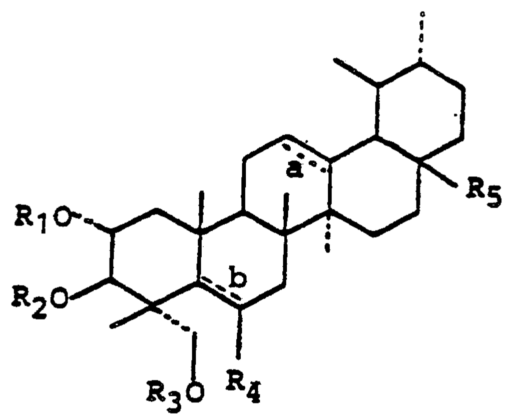

(I′)

in which

$R_1$, $R_2$ and $R_3$ which may be the same or different stand for hydrogen, alkyl, aralkyl, acyl, substituted aminoacyl or mono- or polyglycosyl,

$R_4$ stands for hydrogen, hydroxy, alkoxy, aralkoxy, acyloxy or a mono- or poly-glycosyloxyether group, 'b' symbolizes an optional additional bond between carbon atoms 5 and 6; $R_4$ also may stand for an oxo-group when 'b' does not symbolize an additional bond between C5 and C6,

$R_5$ stands for the group:

$COOR_6$, wherein $R_6$ stands for hydrogen, alkyl, substituted alkyl or mono- or polyglycosyl,

or the group

$CH_2OR_7$ wherein $R_7$ stands for hydrogen, alkyl, substituted alkyl, acyl or mono- or polyglycosyl,

or the group

$CONR_8R_9$ wherein $R_8$ and $R_9$, when they are the same, stand for hydrogen, alkyl or substituted alkyl; when $R_8$ stands for hydrogen, $R_9$ stands for alkyl, substituted alkyl or aryl; when $R_8$ and $R_9$ together with the nitrogen atom to which they are attached form a heterocycle, which may contain more than one heteroatom and is optionally substituted at one or more places by groups such as alkyl, hydroxy, alkoxy, aryl or another heterocyclic group and

'a' stands for an optional additional bond between carbon atoms C12 and C13,

for the treatment or prophylaxis of cognitive disorders, cerebral vascular diseases and diseases of the central nervous system.

When the compounds of formula I or of formula I′ are capable of forming salts, the invention also includes all such pharmaceutically acceptable salts thereof.

The term alkyl relates to straight or branched saturated hydrocarbon radicals having 1 to 8 carbon atoms such as for example methyl, ethyl, propyl, 2-methylpropyl, 1-pentyl, 3-hexyl or 2-octyl and the like. Preferred alkyl groups have 1 to 6, in particular 1 to 4, carbon atoms.

Suitable examples of substituted alkyl groups are hydroxyalkyls such as propylene glycol.

An aralkyl group is to be understood to be a phenylalkyl group. Preferably phenyl-$C_1$-$C_3$-alkyl for example a benzyl group in which the phenyl group is substituted one or more times by halogen, $C_1$-$C_3$-alkyl, $C_1$-$C_3$-alkoxy, hydroxy, nitro, amino or trifluoromethyl.

An aryl group is to be understood to be a phenyl group which can be substituted one or more times by substituents such as halogen, $C_1$-$C_3$-alkyl, $C_1$-$C_3$-alkoxy, hydroxy, nitro, amino or trifluoromethyl.

An acyl group is to be understood as $C_1$-$C_6$-alkanoyl, $C_2$-$C_6$-alkenoyl, $C_3$-$C_6$-alkynoyl, aroyl, aralkanoyl, heteroalkanoyl or a heteroaroyl group having up to 10 carbon atoms, it being possible for one or more carbon atoms to be replaced by oxygen, nitrogen and/or sulphur.

A substituted acyl group is to be understood as chloroacylaminoacyl or heterocyclic acyl such as piperidinoacyl, morpholinoacyl, imidazolinoacyl etc.

Examples of alkanoyl groups are formyl, acetyl, propionyl, butyryl, isobutyryl, valeryl, palmityl and bromoisobutyryl.

The alkenoyl groups can contain one or more double bonds for example an acryloyl, stearyl or oleoyl group. The alkenoyl groups can also contain one or more triple bonds as well as one or more double bonds. An example of alkynoyl group of this type is the propargyl group.

A representative of aroyl groups is the benzoyl group in which the phenyl group is substituted once or several times by substituents such as $C_1$-$C_3$-alkyl, $C_1$-$C_3$-alkoxy, hydroxy, halogen, nitro, amino or trifluoromethyl.

Examples of aralkanoyl and heteroaroyl groups are phenylacetyl and pyridine-3-carbonyl.

Example of heteroalkanoyl groups are 2-pyrrolidone-5-alkanoyls represented by formula A wherein n = 1-3 and 2-azetidinone-4-alkanoyls represented by formula B wherein n = 1-3.

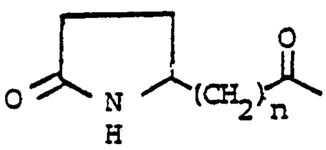

Formula A

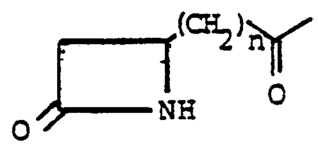

Formula B

Examples of monoglycosyl are glucosyl or rhamnosyl.

Examples of polyglycosyl groups are -glucosyl-rhamnosyl or -glucosyl-rhamnosyl-glucosyl.

Examples of heterocycles are piperidine, morpholine, piperazine, pyrrolidine, thiomorpholine, each of which can optionally be substituted in one or more positions by $C_1$-$C_4$-alkyl, $C_1$-$C_4$-alkoxy, aryl, and aryl-$C_1$-$C_4$-alkyl, hydroxy, amino or substituted $C_1$-$C_4$-alkyl.

Suitable examples of salts of the compounds according to the invention with inorganic or organic acids are the hydrochlorides, hydrobromides, sulphates, phosphates, acetates, oxalates, tartrates, citrates, maleates or fumarates when the compound has a basic group, or salts such as sodium- or potassium-salts when the compound has an acid group.

Specifically, the invention relates to compounds of the formula II

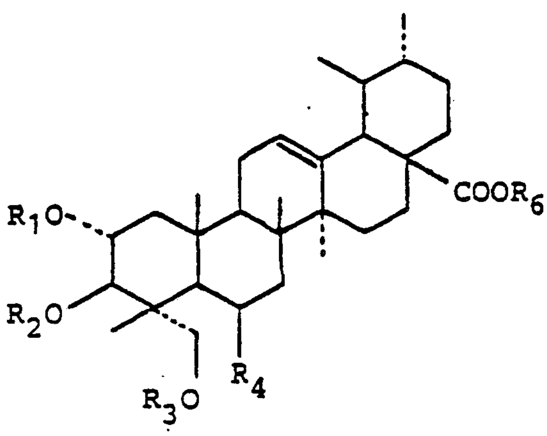

(II),

wherein

$R_1$ - $R_3$ stand for H, $C_1$-$C_6$-alkanoyl, 2 keto-pyrrolidyl-5-alkanoyl or 2-keto-azetidinoyl-alkanoyl,

$R_4$ stands for H or OH and

$R_6$ stands for H, glucosyl, diglucosyl, rhamnosyl or a -glucosyl-glucosyl-rhamnosyl group for the treatment or prophylaxis of cognitive disorders, cerebral vascular diseases and diseases of the central nervous system.

More specifically, the invention relates to the compounds asiaticoside (III), madecassoside (IV), asiatic acid (V) and madecassic acid (VI) and their derivatives which fall under the scope of formulas I and I′ of the invention for the treatment or prophylaxis of cognitive disorders, cerebral vascular diseases and diseases of

the central nervous system.

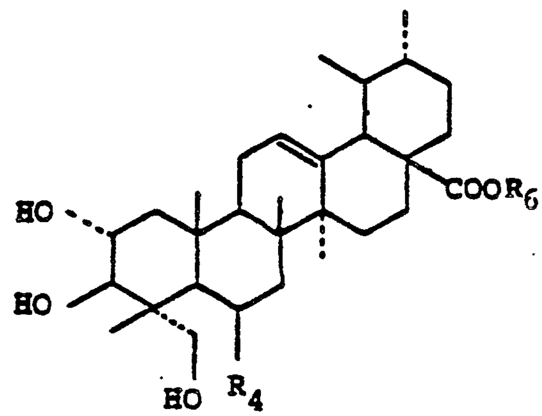

III - VI

Asiaticoside III, $R_4$ = H
$R_6$ = -glu-glu-rham

Asiatic Acid V, $R_4$ = H
$R_6$ = H

Madecassoside IV, $R_4$ = OH
$R_6$ = -glu-glu-rham

Madecassic Acid VI, $R_4$ = OH
$R_6$ = H

Substituent $R_6$ in the compounds of formulae III and IV is preferably a substituent of formula C

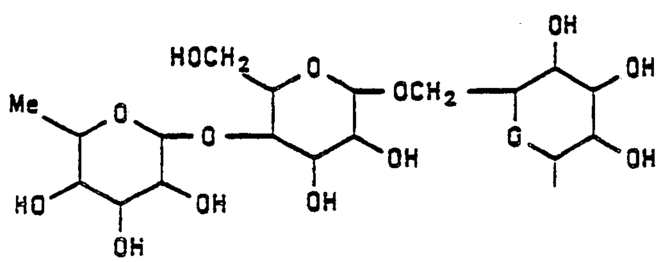

(C)

(a O- 6- deoxy-α- L-mannopyranosyl-(1→4)-O-β-D-glucopyranosyl-(1→6)-β-D-glucopyranosyl residue)

The invention also relates to processes for isolating the compounds from natural sources such as plants and microbial fermentation broths, and to processes for synthesizing the compounds.

Compounds of formula I of the invention wherein $R_1$ - $R_3$ stand for H, $R_4$ stands for H or OH and $R_5$ stands for $COOR_6$ wherein $R_6$ stands for H or triglycoside are known to be constituents of plants such as Stemonoporus oblongifolius, Vateria indica, Dipterocarpus zeylanicus, Dipterocarpus pilosus, Doona congestiflora, Doona macrophylla, Rhododendron japanicum and Centella asiatica. Such compounds are obtained by extraction of these plants and purification of their extracts according to procedures described in the literature, [for instance, Phyto-Chem. 8,917-21 (1969), Boll. Chim. Farm 120 570-605 (1981)] or modifications thereof.

Compounds of formula I, wherein $R_1$ - $R_3$ stand for H, $R_4$ stands for H or OH and $R_5$ stands for COOH [VII] are alternatively prepared by saponification of the corresponding esters of the formula VIII, following known methods described in the literature.

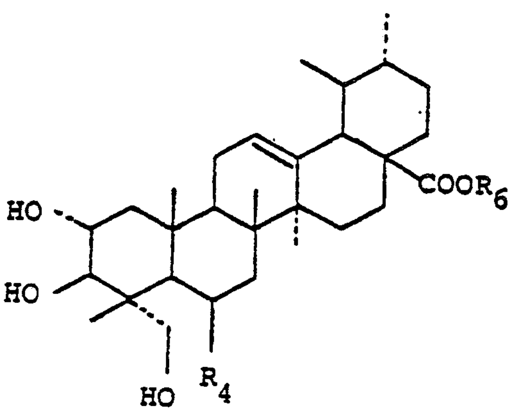

VIII

$R_4$ = H, OH
$R_6$ = alkyl, mono- or polyglycosyl

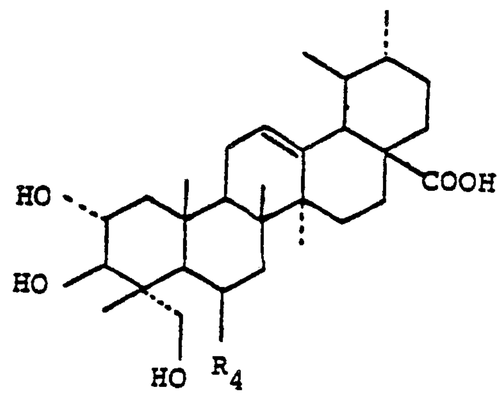

VII

$R_4$ = H, OH

Compounds of formula I wherein $R_1$ - $R_3$ stand for acyl, $R_4$ stands for H and $R_6$ stands for H or mono- or polyglycoside are preferably prepared by acylating compounds of the formula VIII wherein $R_4$ = H, $R_6$ = mono or polyglycoside following the standard methods of acylation described in Reagents for Organic Synthesis by Fieser & Fieser, John Wiley, New York.

Compounds of formula I wherein $R_1$ - $R_3$ stand for H, $R_4$ stands for H or OH, $R_5$ stands for $COOR_6$ and $R_6$ stands for mono- or polyglycosides are preferably prepared by glycosidation of compounds of formula II, wherein $R_1$ - $R_3$ stand for standard hydroxyl protecting groups such as acyl, benzyl etc., $R_4$ stands for H or for a protected hydroxyl group and $R_6$ stands for H by treatment with appropriate halo sugars following the procedures described in the literature [Ref. H. Paulsen Angew. Chem. Int. Ed. Engl., 21, 155 (1982) and R. R. Schmidt, Angew. Chem. Int. Ed. Engl., 25, 212 (1986)] and in particular in the presence of silver carbonate and an aromatic hydrocarbon such as benzene, toluene as solvents and deprotecting the protected hydroxyl groups.

Similarly, compounds of formula I wherein $R_1$ - $R_3$ stand for H and $R_4$ stands for H or OH, $R_5$ stands for $COOR_6$ and $R_6$ stands for alkyl, are preferably prepared from compounds of formula VII in which $R_4$ stands H or OH by esterification with an appropriate alcohol in the presence of an acid such as HCl or $H_2SO_4$ or by treatment with a diazoalkane following known methods described in the literature.

Compounds of the formula I wherein $R_1$ - $R_3$ stand for H, $R_4$ stands for H or OH and $R_5$ stands for $CH_2OH$ are preferably prepared from compounds of formula II, wherein $R_1$-$R_3$ stand for H, $R_4$ stands vor H or OH and $R_6$ stands for an alkyl group, by treatment with a metal hydride such as lithiumaluminiumhydride in organic solvents such as anhydrous diethylether or dioxane or tetrahydrofuran.

Compounds of formula I wherein $R_1$ - $R_3$ stand for H, alkyl, acyl, aralkyl, $R_4$ stands for H, OH, alkoxy, acyloxy or aralkoxy, $R_5$ stands for $COOR_6$ and $R_6$ stands for alkyl, acyl, aralkyl mono- or polyglycoside are preferably prepared from compounds of formula II wherein $R_6$ stands for mono- or polyglycoside, $R_1$ - $R_3$ stand for H and $R_4$ stands for H or OH by alkylation or acylation using known methods described in the literature.

The administration of compounds of the instant invention improves impaired cognitive functions in mammals and thus they are useful drugs for the prevention and treatment of cognitive disorders. They also display inhibitory activity on capillary permeability and serotonin antagonist activity.

The methods adopted for pharmacological evaluation of the compounds of the invention are described below.

Scopolamine-induced dementia in mice

The method to evaluate anti-amnesic activity by Schindler [U. Schindler et al, Drug Dev. Res. 4:567-76 (1984)] with some modification is used.

The following were the modifications: Compound under test is administered once daily for 3 days. On the 4th day, administration is 60 minutes before and 30 minutes after the administration of scopolamine (5 mg/kg, s. c.). AYmA, 2-sec foot shock is applied.

The percentage of the animals showing a significant increase in the latency period of the compound-treated group over the scopolamine-treated group is calculated Statistical significance is analysed by Fisher's test, p value less than 0.05 is chosen as the level of significance.

Table 1

| Effect of Asiaticoside on Scopolamine-induced dementia in mice | | | |
|---|---|---|---|
| Compound | Dose mg/kg p.o. | % Activity | p value |
| Asiaticoside | 15 | 33 | Not significant |
| Asiaticoside | 30 | 92 | < 0.01 |
| Asiaticoside | 60 | 69 | < 0.01 |

Asiaticoside significantly antagonises the scopolamine-induced dementia in mice at 30 and 60 mg/kg p.o. for four days treatment. (Table 1).

Capillary Permeability Test

Charles Foster rats of either sex weighing 120-140 g are shaved on the dorsal side and fasted overnight. After 16-18 hours animals are divided into different groups (6 animals/group) and treated orally either with the vehicle or test drug. One hour later, each group of animals receives intradermally 0.1 ml of 0.9% saline and 0.1 ml of 0.01% (R)Compound 48/80 (condensation product of N-methyl-methoxy-phenethylamine with formaldehyde) or 0.1 ml of 0.05% Bradykinin or 0.01 ml of 0.001% 5-Hydroxy-tryptamine (5-HT) at two different sites. Immediately after intradermal injections, each animal receives 0.2 ml of 1% (R)Evans blue dye intravenously. Animals are sacrificed after 30 minutes and skin is removed from the dorsal side. The leakage of the dye is quantified spectrophotometrically using the method of Kayama [Kayama S. et al Jap. J. Pharmacol. 25, 103 (1975)].

In the present experiment, Asiaticoside at a dose of 200 mg/kg administered orally inhibits Compound 48/80 and 5-Hydroxy tryptamine-induced capillary permeability by 33.7% and 32% respectively. It has no effect on the Bradykinin-induced capillary permeability (Table 2).

Table 2

| Effect of Asiaticoside on the capillary permeability induced by Bradykinin (BK), 5-Hydroxytryptamine (5-HT) and Compound 48/80. | | | | |
|---|---|---|---|---|
| Treatment | Dose p.o. | Dye Concentrations (ug/site) induced by (Mean + S.E.) | | |
| | | BK | 5-HT | 48/80 |
| Vehicle | 10 ml/kg | 13.92 | 32.08 | 42.75 |
| | | ±1.68 | ±3.89 | ±7.73 |
| Asiaticoside | 200 mg/kg | 17.75 | 21.83 | 28.33 |
| | | ±3.55 | ±3.92 | ±7.05 |
| % Inhibition | | 0 | 32 | 33.7 |

Isolated Guinea Pig Ileum

Guinea Pigs of either sex weighing 350-400 g are sacrificed and a piece of ileum is removed. 3 to 4 cm length of ileum is cut, cleaned and mounted in an organ bath containing Tyrode solution. The bath is maintaned at 37° C and aerated with compressed air. Agonist (5-HT and Bradykinin-induced) contractions are measured through K-30 (R)(Hugo Sachs) isometric transducer and recorded on a 4-channel (R)Digilog potentiometric recorder.

Asiaticoside at a dose of 10, 30 and 100 µg/ml shows 10.2, 19.7 and 34.5% inhibition of 5-HT-induced contraction, while Bradykinin- induced contraction of the guinea pig ileum is not affected (Table 3).

Table 3

| Effect of Asiaticoside on isolated guinea pig ileum | | | |
|---|---|---|---|
| Agonists | Concentration µg/ml | Dose of Asiaticoside µg/ml | % Inhibition (Mean + S.E.) |
| Bradykinin | 0.2 - 0.3 | 100 | 0 |
| 5-HT | 2 - 10 | 10 | 10.2 ± 11.9 |
| | | 30 | 19.7 ± 6 |
| | | 100 | 34.5 ± 9 |

The above results indicate that Asiaticoside has an antagonistic effect on serotonergic receptors.

By reason of the actions which have been indicated the compounds of the formulae I and I′ are suitable for the treatment and prophylaxis of cognitive disorders, cerebral vascular diseases and diseases of the central nervous system.

The pharmaceuticals according to the instant invention contain the compounds of the formulae I and I′ as active compound, where appropriate in combination with other active compounds.

The pharmaceuticals are prepared by methods known per se and familiar to the expert. The pharmaceuticals are used in the form of tablets, coated tablets, capsules, suppositories, emulsions, suspensions or solutions with an effective amount of the said active compound either per se or, preferably, in combination with suitable pharmaceutical auxiliaries, the content of active compound being up to about 95 %, preferably between 10 and 75 %. The auxiliaries suitable for the desired medicament formulation are familiar to the expert on the basis of his expert knwoledge. In addition to tableting auxiliaries, solvents, gel-forming agents, suppository bases and other vehicles for active compounds it is possible to use, for example, antioxidants, dispersants, emulsifiers, antifoam agents, flavorings, preservatives, solubilizers or colorants.

The active compound can be administered orally, parenterally, intravenously or rectally, with oral administration being preferred. For a form for oral use, the said active compound is mixed, where appropriate with further active compounds, with the additives suitable for this purpose, such as excipients, stabilizers or inert diluents, and converted by the customary methods into suitable presentations such as tablets, coated tablets, hard gelatin capsules and aqueous, alcoholic or oily suspensions or solutions. Examples of inert vehicles which can be used are gum arabic, magnesia, lactose, glucose or starch, in particular corn starch. Both dry and moist granules can be used for this preparation. Examples of suitable oily excipients or solvents are vegetable or animal oils, such as sunflower oil or fish liver oil.

For subcutaneous or intravenous administration, the active compound is converted into a solution, suspension or emulsion, if desired with the substances customary for this purpose, such as solubilizers, emulsifiers or other auxiliaries. Examples of suitable solvents are water physiological saline solution or alcohols, for example, ethanol, propanol, or glycerol, as well as sugar solutions such as solutions of glucose or mannitol, or a mixture of the various solvents mentioned.

The following examples illustrate the invention but do not limit the scope of the invention.

**Example 1**

Isolation of Asiaticoside

The procedure followed was similar to that described in the literature [Boll. Chim. Farm., 120, 570-605 (1981)] with the modification as described below.

The dried and powdered Centella asiatica plant material (11.5 kgs) was extracted at 45-47° C with 2 X 7 litres of aqueous methanol (90%) for eight hours each time. The Combined extracts were concentrated to 1/10th the volume and allowed to stand at 0° C. The solid which separated was centrifuged and washed with cold water to provide 475 g of dry solid.

The supernatent solution obtained during centrifugation was extracted with n-butanol (3 x 3 l). The butanol extract was washed with 5% NaOH and then with water till the pH of the washings was neutral. The butanol extract was concentrated under reduced pressure to obtain a gummy mass which on trituration with petroleum ether (b.p. 60-80° C) afforded a resinous solid residue of approximately 500 g.

Purification was done by the following two methods :

Method 1

Crystallization of the 500 g crude material using aqueous acetone gave 66 g of asiaticoside. M.p. 230-240° C.

Method 2

Column chromatography over silica gel of 75 g of the crude material yielded 18 g of asiaticoside, m.p. 238-240° C, using methanol:ethylacetate mixture as eluent.

**Example 2**

**Isolation of Madecassoside**

Following the procedure described in Example 1, 500 g of crude material was obtained from 11.5 kg of dried plant material. 55 g of the crude material was column chromatographed on silica gel using chloroform:methanol:water (6.5:3.5:0.5) to obtain 20 g of madecassoside. Recrystallisation of the compound from methanol acetone yielded pure crystals of madecassoside, m.p. 221-223° C.

**Example 3**

**Preparation of Asiatic acid**

A solution of asiaticoside (2.0 g) in methanol (40 ml) was treated with 5N sodium hydroxide solution and the mixture heated at 70-80° C for 2-5 hours. The reaction mixture was then concentrated under vacuo to half of its volume and neutralised with cold 2N hydrochloric acid. The white solid which separated out was filtered and dried to obtain 0.9 g of asiatic acid, m.p. 306-310° C.

**Example 4**

**Preparation of Madecassic acid**

A mixture of madecassoside (4.0 g) and 10% aqueous sodium hydroxide (200 ml) was stirred at 75° C

for 1.5 hours. The reaction mixture was cooled and acidified with dilute hydrochloric acid. The white solid which separated out was filtered and dried to obtain 1.86 g of solid material. Recrystallization of the solid from methanol yielded madecassic acid, m.p. 265-268° C.

**Example 5**

**Acetylation of Asiaticoside**

Acetic anhydride (5 ml) was added to a solution of asiaticoside (0.5 g) in pyridine (5 ml) and the solution was stirred at room temperature for 3.5 hours. The reaction mixture was diluted with ethylacetate, washed with cold 2N hydrochloric acid (30 ml) and brine (dried $Na_2SO_4$), and concentrated in vacuo. The residue was purified by flash chromatography using 1:1 ethylacetate and pet. ether (b.p. 40-60°) as eluent to obtain the polyacetylated compound A (0.275 g) m.p. 150-1° C $[a]_D$ -8.14° [EtOH] and the polyacetylated compound B (0.175 g) m.p. 155-6° C $[a]_D$ -10.9° [EtOH]. By increasing the reaction time from 3.5 hrs to 26 hrs, the polyacetylated compound A could be obtained in an amount of 0.644 g.

**Example 6**

Acetylation of Madecassoside

Polyacetylated madecassoside was obtained following the acetylation procedure described in Example 5 in 90% yield. M.p. 161-162° C.

**Example 7**

Anhydro madecassic acid

A solution of 0.5 g madecassic acid in 25 ml methanol and 10 ml concentrated hydrochloric acid was refluxed for 3 hours. The solvent was evaporated. The residue obtained was suspensed in $H_2O$ and extracted with ethyl acetate. The organic layer was evaporated to dryness and the residue was purified by chromatography over silica gel using chloroform -2 % methanol as eluant. Yield 430 mg, m.p. 182-92° C.

**Example 8**

Madecassic acid methyl ester

Madecassic acid 1.15 g was dissolved in 50 ml methanol and cooled to 0° C. Excess diazomethane in diethyl ether was added till reaction mixture was pale yellow. After keeping for 1 hour at room temperature, the solvent was evaporated to obtain a residue. Crystallization from ethanol afforded the pure compound 1.2 g. m. p. 162-168° C.

Example 9

Asiatic acid methyl ester

Methyl ester of asiatic acid was obtained following the procedure described in Example 8.
m.p. 162 - 166° C

## Claims

1. 2,3,23-Trihydroxy-urs-12-ene derivatives of the formula I

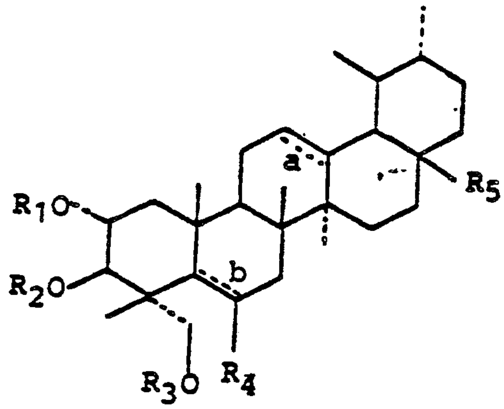

(I)

in which
$R_1$, $R_2$ and $R_3$ which may be the same or different stand for hydrogen, alkyl, aralkyl, acyl, substituted aminoacyl or mono- or polyglycosyl,
$R_4$ stands for hydrogen, hydroxy, alkoxy, aralkoxy, acyloxy or a mono- or polyglycosyloxyether group, 'b' symbolizes an optional additional bond between carbon atoms $C_5$ and $C_6$; $R_4$ also may stand for an oxo-group when 'b' does not symbolize an additional bond between C5 and C6,
$R_5$ stands for the group
$COOR_6$, wherein $R_6$ stands for hydrogen, alkyl, substituted alkyl or mono- or polyglycosyl, or the group
$CH_2OR_7$ wherein $R_7$ stands for hydrogen, alkyl, substituted alkyl, acyl or mono- or polyglycosyl, or the group
$CONR_8R_9$ wherein $R_8$ and $R_9$ when they are the same, stand for hydrogen, alkyl or substituted alkyl; when $R_8$ stands for hydrogen, $R_9$ stands for alkyl, substituted alkyl, aryl; when $R_8$ and $R_9$ together with the nitrogen atom to which they are attached form a heterocycle, which may contain more than one heteroatom and is optionally substituted at one or more places by groups such as alkyl, hydroxy, alkoxy, aryl or another heterocyclic group and
'a' symbolizes an optional double bond between carbon atoms C12 and C13,
with the exception of those compounds, in which at the same time $R_1$, $R_2$ and $R_3$ are H or $R_1$, $R_2$ and $R_3$ are acetyl, $R_4$ is H or OH and $R_5$ is COOH or $COOCH_3$ or in which at the same time $R_1$ and $R_3$ are acetyl, $R_2$ is H or $R_1$ is acetyl and $R_2$ and $R_3$ are H, $R_4$ is H and $R_5$ is COOH or $COOCH_3$ or in which at the same time $R_1$, $R_2$ and $R_3$ are H, $R_4$ is H or OH and $R_5$ is a O-6-deoxy-α-L-mannopyranosyl-(1→4)-O-β-D-glucopyranosyl-(1→6)-β-D-glucopyranosyl-oxycarbonyl-residue or in which at the same time $R_1$, $R_2$ and $R_4$ are H, $R_2$ is $CH_3$ and $R_5$ is $COOCH_3$.

2. 2,3,23-Trihydroxy-urs-12-ene and its derivatives of the formula I′

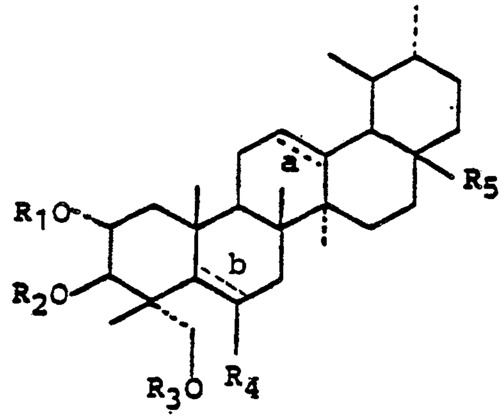

(I')

in which
$R_1$, $R_2$ and $R_3$ which may be the same or different stand for hydrogen, alkyl, aralkyl, acyl, substituted aminoacyl or mono- or polyglycosyl,
$R_4$ stands for hydrogen, hydroxy, alkoxy, aralkoxy, acyloxy or a mono- or polyglycosyloxyether group, 'b' symbolizes an optional additional bond between carbon atoms C5 and C6; $R_4$ also may stand for an oxo-group when 'b' does not symbolize an additional bond between C5 and C6,
$R_5$ stands for the group
$COOR_6$, wherein $R_6$ stands for hydrogen, alkyl, substituted alkyl or mono- or polyglycosyl,
or the group:
$CH_2OR_7$ wherein $R_7$ stands for hydrogen, alkyl, substituted alkyl, acyl or mono- or polyglycosyl,
or the group:
$CONR_8R_9$ wherein $R_8$ and $R_9$, when they are the same, stand for hydrogen, alkyl or substituted alkyl; when $R_8$ stands for hydrogen, $R_9$ stands for alkyl, substituted alkyl or aryl; when $R_8$ and $R_9$ together with the nitrogen atom to which they are attached form a heterocycle, which may contain more than one heteroatom and is optionally substituted at one or more places by groups such as alkyl, hydroxy, alkoxy, aryl or another heterocyclic group and
'a' symbolizes an optional additional bond between carbon atoms C12 and C13,
for the treatment or prophylaxis of cognitive disorders, cerebral vascular diseases and diseases of the central nervous system.

3. 2,3,23-Trihydroxy-urs-12-ene derivatives as claimed in claim 2, characterized by the formula II

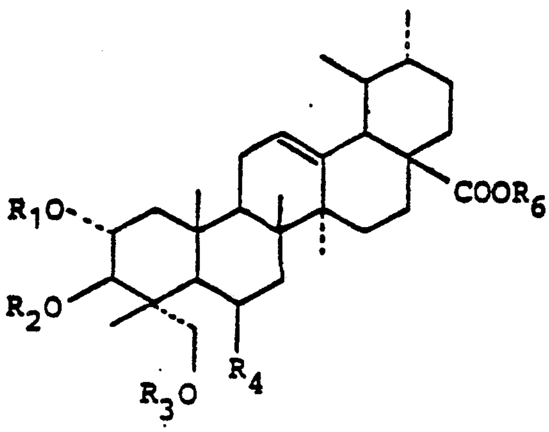

(II),

wherein
$R_1$ - $R_3$ stand for H, $C_1$-$C_6$-alkanoyl, 2 keto-pyrrolidyl-5-alkanoyl or 2-keto-azetidinoyl-alkanoyl,
$R_4$ stands for H or OH and
$R_6$ stands for H, glucosyl, diglucosyl, rhamnosyl or a -glucosyl-glucosyl-rhamnosyl group and the pharmaceutically acceptable salts thereof for the treatment or prophylaxis of cognitive disorders, cerebral vascular diseases and diseases of the central nervous system.

4. 2,3,23-Trihydroxy-urs-12-ene of the formula II as claimed in claim 3, wherein $R_1$, $R_2$ and $R_3$ are H, $R_4$ is H or OH and $R_6$ is H or a -glucosyl-glucosyl-rhamnosyl group for the treatment or prohpylaxis of cognitive disorders, cerebral vascular diseases and diseases of the central nervous system.

5. A pharmaceutical which contains at least one of the compounds of formula I as claimed in claim 1.

6. A pharmaceutical which contains at least one of the compounds of formula I′ or of formula II as claimed in claims 2 - 4 for the treatment or the prophylaxis of cognitive disorders, cerebral vascular diseases and diseases of the central nervous system.

7. The use of a compound of formula I, formula I′ or formula II as claimed in claims 1 - 4 for the treatment or the prophylaxis of cognitive disorders, cerebral vascular diseases and diseases of the central nervous system.

8. The use of a compound of formula I′ or of formula II as claimed in claims 2 - 4 for the production of a pharmaceutical for the treatment or the prophylaxis of cognitive disorders, cerebral vascular diseases and diseases of the central nervous system.

9. A process for the production of compounds of the formula as claimed in claim 1, wherein compounds of formula I in which $R_1$ - $R_3$ stand for acyl, $R_4$ stands for H and $R_5$ stands for $COOR_6$ in which $R_6$ stands for mono- or polyglycoside are prepared by acylating compounds of the formula VIII

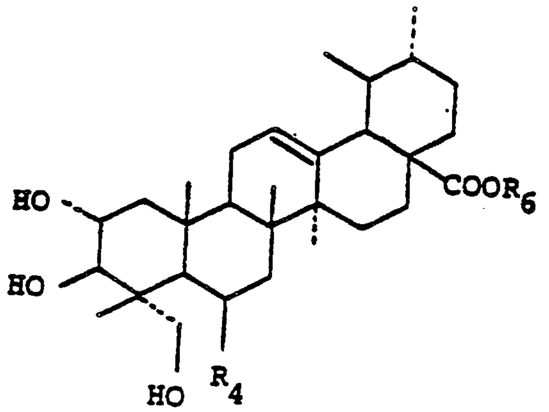

(VIII)

wherein $R_4$ is H
and $R_6$ is mono- or polyglycoside
in a manner known per se,
or compounds of formula I wherein $R_1$ - $R_3$ stand for H, $R_4$ stands for H or OH and $R_5$ stands for $COOR_6$ and $R_6$ stands for mono- or polyglycoside are prepared by glycosidation of compounds of formula II, wherein $R_1$ -$R_3$ stand for standard hydroxyl protecting groups, $R_4$ stands for H or for a protected hydroxyl group and $R_6$ stands for H, by treatment with appropriate halosugars in a manner known per se and deprotecting the protected hydroxyl groups,
or compounds of formula I, wherein $R_1$ - $R_3$ stand for H, $R_4$ stands for H or OH, $R_5$ stands for $COOR_6$ and $R_6$ stands for alkyl,
are prepared from compounds of formula VII

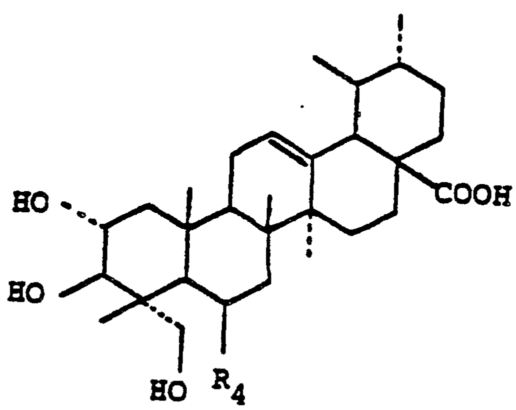

(VII)

in which $R_4$ denotes H or OH, by esterification with an appropriate alcohol in the presence of an acid in a manner known per se or compounds of the formula I wherein $R_1$ - $R_3$ stand for H, $R_4$ stands for H or OH and $R_5$ stands for $CH_2OH$ are prepared from compounds of formula II, wherein $R_1$-$R_3$ stand for H, $R_4$ stands for H or OH and $R_6$ stands for an alkyl group, by treatment with a metal hydride, or compounds of formula I, wherein $R_1$ - $R_3$ stand for H, alkyl, acyl or aralkyl, $R_4$ stands for H, OH, alkoxy, acyloxy or aralkoxy, $R_5$ stands for $COOR_6$ and $R_6$ stands for alkyl, acyl, aralkyl or mono- or polyglycoside are prepared from compounds of formula II wherein $R_6$ stands for mono- or polyglycoside and $R_1$ - $R_3$ stand for H and $R_4$ stands for H or OH by alkylation or acylation in a manner known per se.

10. A process for the production of a pharmaceutical for the treatment or the prophylaxis of cognitive disorders, cerebral vascular diseases and diseases of the central nervous system, wherein 2,3,23-trihydroxy-urs-12-ene or at least one of its derivatives as claimed in claims 1 - 4 is, optionally together with pharmaceutical acceptable excipients, transformed into a form suitable for administration.

Claims for the following Contracting States: ES,GR

1. A process for the production of 2,3,23-Trihydroxy-urs-12-ene derivatives of the formula I

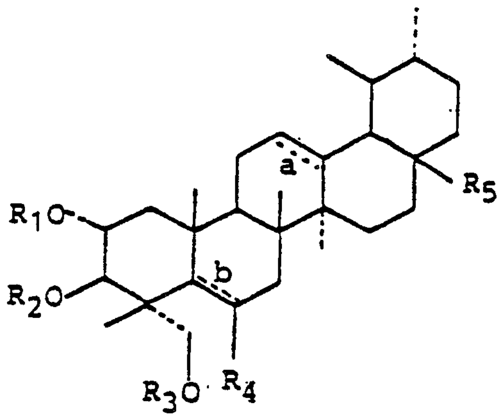

(I)

in which 'a' symbolizes an additional bond between carbon atoms C12 and C13 and 'b' symbolizes no additional bond between carbon atoms C5 and C6, and in which $R_1$ - $R_3$ stand for acyl, $R_4$ stands for H and $R_5$ stands for $COOR_6$ in which $R_6$ stands for mono- or polyglycoside, are prepared by acylating compounds of the formula VIII

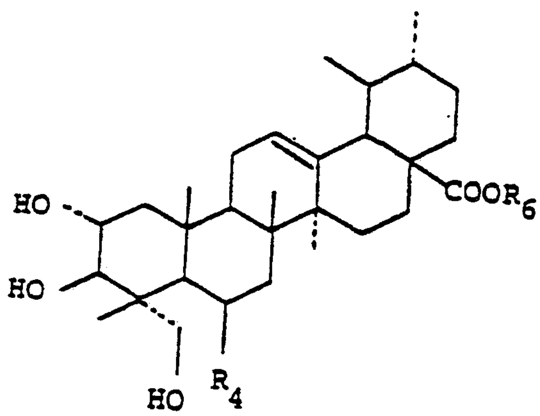

(VIII)

wherein $R_4$ is H
and $R_6$ is mono- or polyglycoside
in a manner known per se,
or compounds of formula I wherein $R_1$ - $R_3$ stand for H, $R_4$ stands for H or OH and $R_5$ stands for $COOR_6$ and $R_6$ stands for mono- or polyglycoside are prepared by glycosidation of compounds of formula II,

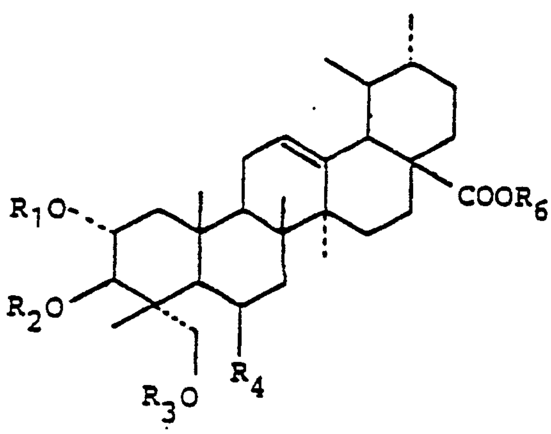

(II)

wherein $R_1$ - $R_3$ stand for standard hydroxyl protecting groups, $R_4$ stands for H or for a protected hydroxyl group and $R_6$ stands for H, by treatment with appropriate halosugars in a manner known per se and

deprotecting the protected hydroxyl groups,
or compounds of formula I, wherein $R_1$ - $R_3$ stand for H, $R_4$ stands for H or OH, $R_5$ stands for $COOR_6$ and $R_6$ stands for alkyl,
are prepared from compounds of formula VII

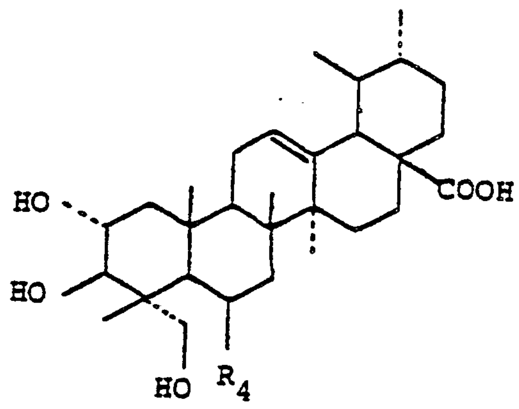

(VII)

in which $R_4$ denotes H or OH, by esterification with an appropriate alcohol in the presence of an acid in a manner known per se or compounds of the formula I wherein $R_1$ - $R_3$ stand for H, $R_4$ stands for H or OH and $R_5$ stands for $CH_2OH$ are prepared from compounds of formula II, wherein $R_1$-$R_3$ stand for H, $R_4$ stands for H or OH and $R_6$ stands for an alkyl group, by treatment with a metal hydride, or compounds of formula I, wherein $R_1$ - $R_3$ stand for H, alkyl, acyl or aralkyl, $R_4$ stands for H, OH, alkoxy, acyloxy or aralkoxy, $R_5$ stands for $COOR_6$ and $R_6$ stands for alkyl, acyl, aralkyl or mono- or polyglycoside are prepared from compounds of formula II wherein $R_6$ stands for mono- or polyglycoside and $R_1$ - $R_3$ stand for H and $R_4$ stands for H or OH by alkylation or acylation in a manner known per se.

2. 2,3,23-Trihydroxy-urs-12-ene and its derivatives of the formula I′

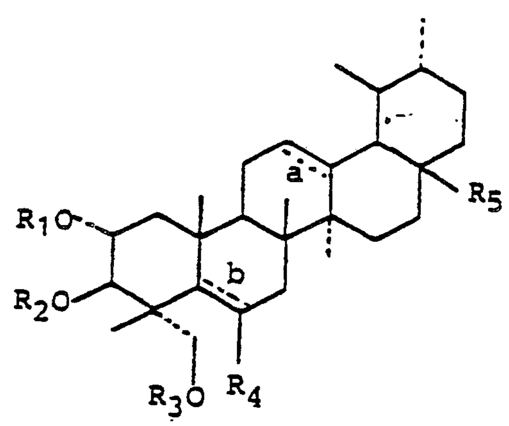

(I′)

in which
$R_1$, $R_2$ and $R_3$ which may be the same or different stand for hydrogen, alkyl, aralkyl, acyl, substituted aminoacyl or mono- or polyglycosyl,
$R_4$ stands for hydrogen, hydroxy, alkoxy, aralkoxy, acyloxy or a mono- or polyglycosyloxyether group, 'b' symbolizes an optional additional bond between carbon atoms C5 and C6; $R_4$ also may stand for an oxo-group when 'b' does not symbolize an additional bond between C5 and C6, $R_5$ stands for the group
$COOR_6$, wherein $R_6$ stands for hydrogen, alkyl, substituted alkyl or mono- or polyglycosyl,
or the group:
$CH_2OR_7$ wherein $R_7$ stands for hydrogen, alkyl, substituted alkyl, acyl or mono- or polyglycosyl, or the group:
$CONR_8R_9$ wherein $R_8$ and $R_9$, when they are the same, stand for hydrogen, alkyl or substituted alkyl; when $R_8$ stands for hydrogen, $R_9$ stands for alkyl, substituted alkyl or aryl; when $R_8$ and $R_9$ together with the nitrogen atom to which they are attached form a heterocycle, which may contain more than one heteroatom and is optionally substituted at one or more places by groups such as alkyl, hydroxy, alkoxy, aryl or another

heterocyclic group and
'a' symbolizes an optional additional bond between carbon atoms C12 and C13,
for the treatment or prophylaxis of cognitive disorders, cerebral vascular diseases and diseases of the central nervous system.

3. 2,3,23-Trihydroxy-urs-12-ene derivatives as claimed in claim 2, characterized by the formula II

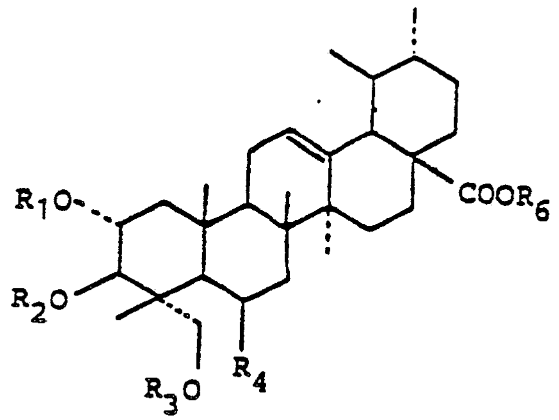

(II),

wherein
$R_1$ - $R_3$ stand for H, $C_1$-$C_6$-alkanoyl, 2 keto-pyrrolidyl-5-alkanoyl or 2-keto-azetidinoyl-alkanoyl,
$R_4$ stands for H or OH and
$R_6$ stands for H, glucosyl, diglucosyl, rhamnosyl or a -glucosyl-glucosyl-rhamnosyl group and the pharmaceutically acceptable salts thereof for the treatment or prophylaxis of cognitive disorders, cerebral vascular diseases and diseases of the central nervous system.

4. 2,3,23-Trihydroxy-urs-12-ene of the formula II as claimed in claim 3, wherein $R_1$, $R_2$ and $R_3$ are H, $R_4$ is H or OH and $R_6$ is H or a -glucosyl-glucosyl-rhamnosyl group for the treatment or prohpylaxis of cognitive disorders, cerebral vascular diseases and diseases of the central nervous system.

5. A pharmaceutical which contains at least one of the compounds of formula I as claimed in claim 1.

6. A pharmaceutical which contains at least one of the compounds of formula I′ or of formula II as claimed in claims 2 - 4 for the treatment or the prophylaxis of cognitive disorders, cerebral vascular diseases and diseases of the central nervous system.

7. The use of a compound of formula I, formula I′ or formula II as claimed in claims 1 - 4 for the treatment or the prophylaxis of cognitive disorders, cerebral vascular diseases and diseases of the central nervous system.

8. The use of a compound of formula I′ or of formula II as claimed in claims 2 - 4 for the production of a pharmaceutical for the treatment or the prophylaxis of cognitive disorders, cerebral vascular diseases and diseases of the central nervous system.

9. A process for the production of a pharmaceutical for the treatment or the prophylaxis of cognitive disorders, cerebral vascular diseases and diseases of the central nervous system, wherein 2,3,23-trihydroxy-urs-12-ene or at least one of its derivatives as claimed in claims 1 - 4 is, optionally together with pharmaceutical acceptable excipients, transformed into a form suitable for administration.